(19) Europäisches Patentamt
European Patent Office
Office européen des brevets

(11) Veröffentlichungsnummer: **0 338 308**
**A2**

(12) **EUROPÄISCHE PATENTANMELDUNG**

(21) Anmeldenummer: **89105797.8**

(51) Int. Cl.4: **C07H 15/12 , A61K 31/70**

(22) Anmeldetag: **03.04.89**

(30) Priorität: **16.04.88 DE 3812681**

(43) Veröffentlichungstag der Anmeldung:
**25.10.89 Patentblatt 89/43**

(84) Benannte Vertragsstaaten:
**AT BE CH DE ES FR GB GR IT LI NL SE**

(71) Anmelder: **BAYER AG**

**D-5090 Leverkusen 1 Bayerwerk(DE)**

(72) Erfinder: **Lockhoff, Oswald, Dr.**
**Morgengraben 14**
**D-5000 Köln 80(DE)**
Erfinder: **Goossens, John, D.I.**
**Hebbelstrasse 5**
**D-5090 Leverkusen 1(DE)**
Erfinder: **Brunner, Helmut, Prof. Dr.**
**Am Rohm 111**
**D-5600 Wuppertal 1(DE)**
Erfinder: **Sperzel, Michael, Dr.**
**Siegfriedstrasse 39**
**D-5600 Wuppertal 1(DE)**
Erfinder: **Stünkel, Klaus Georg, Dr.**
**Am Eckbusch 55/12**
**D-5600 Wuppertal 1(DE)**

(54) **Substituierte N-Glycosylamide, Verfahren zu ihrer Herstellung sowie ihre Verwendung als Arzneimittel.**

(57) Die Erfindung betrifft neue N-Glycosylamide der allgemeinen Formel I

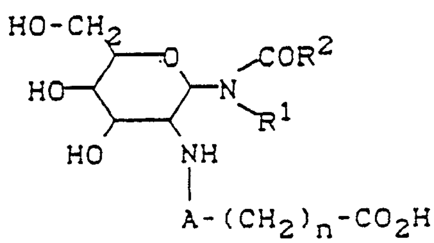

in der
$R^1$ einen gesättigten Alkylrest oder einen einfach, zweifach oder dreifach ungesättigten Alkenylrest mit bis zu 50 Kohlenstoffatomen bedeutet,
$R^2$ einen gesättigten Alkylrest oder einen einfach, zweifach oder dreifach ungesättigten Alkenylrest mit bis zu 50 Kohlenstoffatomen bedeutet,
A für -CO- oder für eine Gruppe

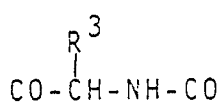

steht, worin
$R^3$ für Wasserstoff, $C_1$-$C_7$-Alkyl, Hydroxymethyl, 1-Hydroxyethyl, Mercaptomethyl, 2- Methylthio -ethyl, 3-Aminopropyl, 3-Ureidopropyl, 3-Guanidylpropyl, 4-Aminobutyl, Carboxymethyl, Carbamoylmethyl, 2-Car-

EP 0 338 308 A2

boxyethyl, 2-Carbamoylethyl, Benzyl, 4-Hydroxybenzyl, 3-Indolylmethyl oder 4-Imidazolylmethyl steht und
n für 0 bis 10 steht,
und die im Zuckerrest mit einem Aminosäurerest substituiert sind, sowie Verfahren zu ihrer Herstellung und ihre Verwendung als Arzneimittel.

## Substituierte N-Glycosylamide, Verfahren zu ihrer Herstellung sowie ihre Verwendung als Arzneimittel

Die Erfindung betrifft neue N-Glycosylamide, die im Zuckerrest mit einem Aminosäurerest substituiert sind, sowie Verfahren zu ihrer Herstellung und ihre Verwendung als Arzneimittel.

Es ist bereits bekannt, daß die Verbindungsklasse der N-Glycosylamide, N-Glycosylharnstoffe und N-Glycosylcarbamate, die im Zuckerrest mit einer Aminosäure substituiert sind, die Antikörpersynthese des Immunsystems bei Stimulation mit einem Antigen steigern und darüber hinaus unspezifisch die wirtseigene Abwehr verstärken.

Einzelheiten über die Chemie und auch die Wirkungen dieser Verbindungen sind in der DE-OS A 2 206 037 und auch in der EP-OS B 1 091 645 beschrieben.

Ein Nachteil der dort beschriebenen Verbindungen aber ist, daß sie hydrophob sind und dadurch in Wasser allein praktisch nicht formulierbar sind.

Versuche, die Wasserlöslichkeit durch chemische Derivatisierungen zu verbessern, führten bis jetzt immer zu einer Reduktion der Wirkung.

Es wurde nun überraschenderweise festgestellt, daß die im folgenden genannten Verbindungen unter Erhalt der Wirkung eine gute Wasserlöslichkeit besitzen.

Die neuen Verbindungen entsprechen der allgemeinen Formel I

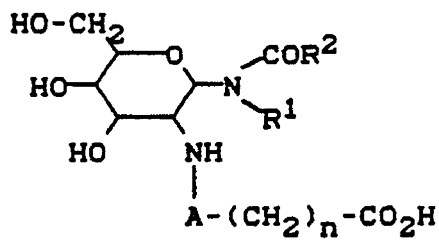

in der
$R^1$ einen gesättigten Alkylrest oder einen einfach, zweifach oder dreifach ungesättigten Alkenylrest mit bis zu 50 Kohlenstoffatomen bedeutet
$R^2$ einen gesättigten Alkylrest oder einen einfach, zweifach oder dreifach ungesättigten Alkenylrest mit bis zu 50 Kohlenstoffatomen bedeutet,
A für -CO- oder für eine Gruppe

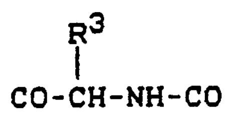

steht,
worin
$R^3$ für Wasserstoff, $C_1$-$C_7$-Alkyl, Hydroxymethyl, 1-Hydroxymethyl, Mercaptomethyl, 2-Methylthioethyl, 3-Aminopropyl, 3-Ureidopropyl, 3-Cuanidylpropyl, 4-Aminobutyl, Carboxymethyl, Carbamoylmethyl, 2-Carboxyethyl, 2-Carbamoylethyl, Benzyl, 4-Hydroxybenzyl, 3-Indolylmethyl oder 4-Imidazolylmethyl steht und
n für 0 bis 10 steht.

$R^1$ und $R^2$ stellen bevorzugt einen geradkettigen oder verzweigten, gesättigten Alkylrest oder einen einfach, zweifach oder dreifach ungesättigten Alkenylrest mit bis zu 30 Kohlenstoffatomen dar. Besonders bevorzugt stehen $R^1$ und $R^2$ für einen Rest mit 8 bis 20 Kohlenstoffatomen.

Besonders bevorzugte Beispiele für Alkylreste in $R^1$ oder $R^2$ sind Decyl, Undecyl, Dodecyl, Tridecyl, Tetradecyl, Pentadecyl, Hexadecyl, Heptadecyl, Octadecyl, Nonadecyl und Eicosyl.

Beispiele für die besonders bevorzugten ungesättigten Alkenylreste von $R^1$ oder $R^2$ sind Decenyl, Undecenyl, Dodecenyl, Tridecenyl, Tetradecenyl, Pentadecenyl, Hexadecenyl, Heptadecenyl, Octadecenyl, Nonadecenyl und Eicosenyl.

Die Reste $R^1$ können auch verzweigte, gesättigte Alkylketten oder einfach oder zweifach ungesättigte Alkenylketten sein. Hierbei sind als Alkylsubstituenten solche Alkylreste besonders bevorzugt, die bis zu 12 Kohlenstoffatome aufweisen.

Der Rest $R^2$ stellt bevorzugt einen geradkettigen oder verzweigten, gesättigten oder einen einfach, zweifach oder dreifach ungesättigten Alkylrest mit bis zu 30 Kohlenstoffatomen dar, besonders bevorzugt mit 8 bis 20 Kohlenstoffatomen.

Bevorzugt für $R^3$ stehen Wasserstoff, $C_1$-$C_7$-Alkyl, Hydroxymethyl, 1-Hydroxyethyl, Carboxymethyl, 2-Carboxyethyl, Benzyl und 4-Hydroxybenzyl.

$R^3$ steht besonders bevorzugt für Wasserstoff oder $C_1$-$C_7$-Alkyl.

Der Umfang der Erfindung erfaßt auch Salze der Verbindungen der Formel I. Dabei handelt es sich in erster Linie um üblicherweise pharmazeutisch verwendbare, nicht toxische Salze der Verbindungen der Formel I. Erhältlich sind diese Salze durch Umsetzung mit geeigneten Basen. Beispiele herfür sind Äthanolamin, Äthylendiamin, Amonium, Cholin, Calcium, Dicyclohexylamin, Diisopropylamin, Kalium, Magnesium, N-Methylglucamin, Natrium, Morpholin, Piperazin, Piperidin oder Tris-(hydroxymethyl)aminomethan.

Wie in Formel I ersichtlich ist, liegt den erfindungsgemäßen Verbindungen als Grundstruktur eine substituierte 2-Amino-2-desoxyhexose zugrunde. Diese Zucker sind immer über das anomere Kohlenstoffatom N-glycosidisch mit der Alkylamidogruppierung mit den oben angegegeben Bedeutungen für $R^1$ und $R^2$ verbunden.

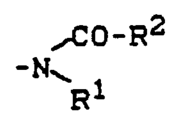

Bevorzugte Aminozucker in den erfindungsgemäßen Verbindungen der Formel I sind 2-Amino-2-desoxy-D-glucose und 2-Amino-2-desoxy-D-galactose.

Die 2-Aminogruppe der genannten Aminozucker in den erfindungsgemäßen Verbindungen der Formel I ist entweder amidisch mit einem Säurerest $-CO-(CH_2)_n-CO_2H$ oder amidisch mit einem α-Acylaminocarbonsäurederivat verbunden.

Bevorzugte Aminosäuren sind die natürlichen L-Aminosäuren wie Glycin, Sarcosin, Hippursäure, Alanin, Valin, Leucin, Isoleucin, Serin, Threonin, Cystein, Methionin, Ornithin, Citrullin, Arginin, Asparginsäure, Asparagin, Glutaminsäure, Glutamin, Phenylalanin, Tyrosin, Prolin, Tryptophan, Histidin. Die D-Isomere der genannten Aminosäuren können ebenfalls als Substituenten fungieren.

Die erfindungsgemäßen N-Glycosylamide der allgemeinen Formel (I) können hergestellt werden, indem man Glycosylamide der allgemeinen Formel (II)

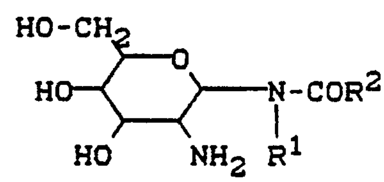

II,

in welcher

$R^1$ und $R^2$ die oben angegebene Bedeutung haben

oder

Peptidoglycolipide der allgemeinen Formel (III),

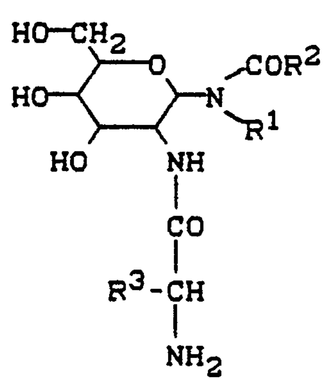

(III)

in der
$R^1$, $R^2$ und $R^3$ die oben angegebene Bedeutung haben,
nach bekannten Methoden mit gegebenenfalls aktivierten Dicarbonsäurederivaten umsetzt, wobei die Aminogruppe in eine Amidogruppe übergeführt wird.

Bei der Variante A werden Verbindungen der allgemeinen Formel (II) oder (III) entweder mit einer nicht aktivierten Dicarbonsäure oder einem Derivat der Dicarbon säure in Gegenwart eines Kondensationsmittels oder aber mit einem aktivierten Dicarbonsäurederivat ohne Gegenwart eines Kondensationsmittels umgesetzt. Die Dicarbonsäurederivate, die in dieser Variante A eingesetzt werden können, werden durch die allgemeine Formel (IV)

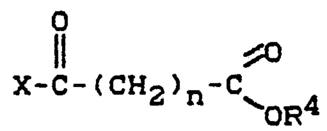
IV

wiedergegeben, in welcher
$R^4$ für Wasserstoff, $C_1$-$C_7$-Alkyl, Aralkyl oder einen gegebenenfalls im Ring substituierten Aralkylrest und
X für Hydroxyl, Alkyloxy, Halogen, Alkylcarbonyloxy, Alkyloxycarbonyloxy oder einen aus der Peptidchemie bekannten Aktivierungsrest wie z.B. Succinimidoxy oder Benzotriazoloxy und
n für 0 - 10 stehen.

Kondensationsmittel werden für den Fall verwendet, wenn X für Hydroxyl steht. Diese Kondensationsmittel sind bekannt, es können z.B. Carbodiimide wie Dicyclohexylcarbodiimid oder Diisopropylcarbodiimid verwendet werden.

Bevorzugte Verbindungen der Formel (IV) sind diejenigen, in denen
$R^4$ für Hydroxyl oder eine $C_1$-$C_3$-Alkylgruppe steht und
X für Halogen oder $C_1$-$C_3$-Alkyloxy steht.

In dem Fall, in dem $R^4$ in den Dicarbonsäurederivaten der Formel IV ungleich Wasserstoff ist, wird der Rest $R^4$ zur Darstellung der Verbindungen der allgemeinen Formel (I) unter basischen oder sauren, bevorzugt basischen Bedingungen hydrolysiert.

Besonders bevorzugt sind die Verbindungen der Formel (IV), in welcher
$R^3$ für Methyloxy oder Ethyloxy und
in der
$R^4$ für eine Hydroxylgruppe
steht.

Diese besonders bevorzugten Verbindungen der Formel IV reagieren mit den Aminen der Formel II oder III unter Ausbildung einer Amidbindung, wobei eine Reaktion abläuft, die in der organischen Chemie als Aminolyse eines Carbonsäureesters bekannt ist.

Bei der Variante B werden Verbindungen der allgemeinen Formeln (II) und (III) mit cyclischen Anhydriden der Formel (V),

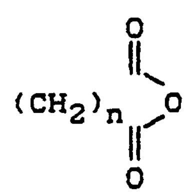
V

in welcher
n vorzugsweise für 2 oder 3 steht,
zu den erfindungsgemäßen Verbindungen der allgemeinen Formel (I) umgesetzt.

Die erfindungsgemäßen Verfahrensvarianten A und B zur Herstellung der Verbindungen der Formel (I) lassen sich schematisch wie folgt darstellen:

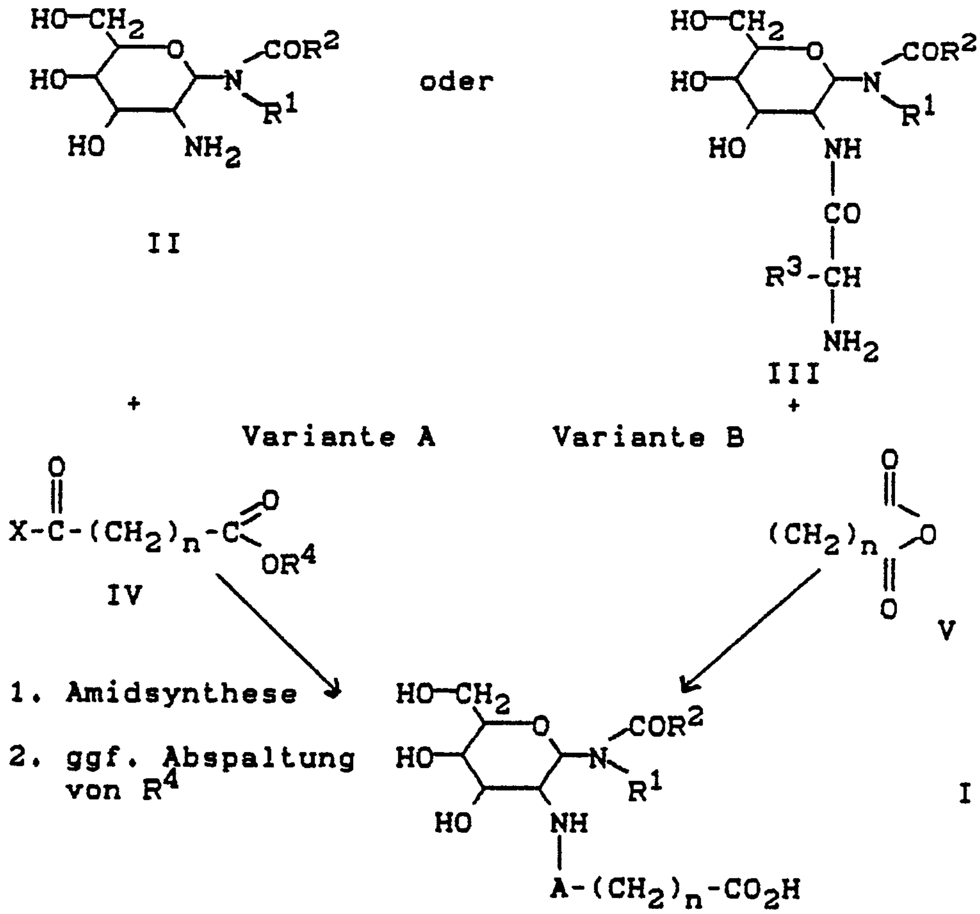


II oder III

IV

V

I

$R^1$, $R^2$, $R^3$ und $R^4$, X und n haben die oben genannte Bedeutung.
A steht entweder für die Gruppe -CO- oder für einen Rest

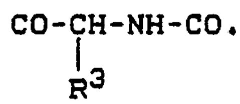

Für das Verfahren kommen als Verdünnungsmittel organische, polare Lösungsmittel in Frage. Hierzu gehören beispielsweise Methanol, THF, DMF und Dioxan.

Im allgemeinen arbeitet man im Fall der Verfahrensvariante A bei einer Reaktionstemperatur um 50°C, bei der Verfahrensvariante B bei Raumtemperatur.

Das Mengenverhältnis der Reaktanden zueinander wird in einigen der hinten beschriebenen Beispielen variiert.

Im allgemeinen werden äquimolare Mengen eingesetzt. Bei der Variante A hat es sich als zweckmäßig erwiesen, die Dicarbonsäurederivate im bis 1,5-fachen Molverhältnis einzusetzen.

Die Verbindungen der Formeln II + III sind bereits bekannt oder können nach bekannten Methoden hergestellt werden (vgl. EP-A 091 645 und EP-A 206 037).

Die vorliegende Erfindung betrifft auch Mittel, die die körpereigene Abwehr verbessern. Es ist üblich, Impfstoffe zusammen mit Adjuvantien zu verabreichen, d.h. mit Substanzen, die die Antikörperbildung verstärken. Für diesen Zweck wird zum Beispiel Freund'sches komplettes Adjuvans eingesetzt. Es handelt sich dabei um eine Wasser-in-Öl-Emulsion, der abgetötete Mykobakterien beigefügt worden sind. Außer der Antikörperbildung können die abgetötete Mykobakterien die zell-vermittelte Immunität und die Makrophagentätigkeit stimulieren.

Es wurde nun gefunden, daß die erfindungsgemäßen Verbindungen der allgemeinen Formel I, deren Definitionen oben aufgeführt sind, die unspezifische Abwehr gegenüber Infektionen steigern.

Die Verbindungen weisen breite Abwehr-stimulierende Wirkungen auf.

Substanzen, die die körpereigene Abwehr (Immunsystem, Phagozytose), während einer Infektion

stimulieren, sind sowohl für die Human- wie für die Veterinärmedizin von großem Interesse, da viele Infektionen trotz guter chemotherapeutischer Möglichkeiten ohne Unterstützung durch körpereigene Abwehrmechanismen persistieren. Dies kann zum erneuten Auftreten von Symptomen (Rezidiv) nach dem Überstehen der Ersterkrankung und damit zu chronisch rezidivierenden Krankheiten führen. Unter den Bakterienbedingten Erkrankungen sind es besonders Infektionen mit fakultativ intrazellulären Bakterien, die Probleme darstellen. Ein Experimentalmodell für eine solche Erkrankung stellt die Infektion der Maus mit Salmonella typhimurium dar. Nach Inokulation der Mäuse mit diesen human- pathogenen Bakterien kommt es in Abhängigkeit von der Infektionsdosis zu einem subakut bis chronischen Krankheitsverlauf, bei dem die Tiere erst nach 4 bis 7 Tagen abzusterben beginnen. Während dieses Zeitraumes besteht die Möglichkeit, das Immunsystem durch Substanzen zu beeinflussen. Während der ersten zwei Wochen werden hohe Keimzahlen im Blut, in der Leber und in der Milz infizierter Tiere gefunden. Die Keimzahlen nehmen danach allmählich ab, sind aber noch 8-12 Wochen nach der Inokulation nachweisbar.

Folgende Wirkungen wurden experimentell festgestellt:

## 1. Wirkungen auf die Keimzahlen in Blut und Leber

Verbindung Beispiel 13 g

Die Verbindung 13 g wurde Mäusen einmalig intraperitoneal in unterschiedlichen Dosen am Tage vor der intraperitonealen Infektion mit 2 x $10^5$ Kolonienbildenden Einheiten (KbE) von Salmonella typhimurium verabfolgt. Diese Infektionsdosis führt bei unbehandelten Tieren am 3. Tag zu einer hohen Keimzahl im Blut und in den Organen, insbesondere in Leber und Milz.

In mehreren Versuchen (s. Tabelle 1) kam es nach der Behandlung der Tiere mit 1 mg/kg oder 10 mg/kg Wirkstoff zu einer deutlichen Verminderung der Keimzahlen infizierter Mäuse im Blut und in der Leber im Vergleich zu Tieren, die nur Verdünnungsmittel (Kontrolle) erhalten hatten. Da die Substan zen in vitro keinen direkten Effekt auf die Vermehrung von Salmonella typhimurium zeigen, sind die Ergebnisse ein Anzeichen dafür, daß Abwehrmechanismen des Wirtes gesteigert werden.

Tabelle 1

| Wirkung von der Verbindung Beispiel 13 g auf die Keimzahlen in Blut und Leber | | |
|---|---|---|
| Dosis a) (mg/kg) | b) KbE/ml Blut | KbE/g Leber |
| Kontrolle | $10^{3,5 \pm 1,9}$ | $10^{6,9 \pm 1,5}$ |
| 1 | $10^{1,7 \pm 0,9}$ | $10^{4,7 \pm 2,0}$ |
| 10 | $10^{1,2 + 0,6}$ | $10^{5,6 + 0,5}$ |

a) einmal 24 h vor der Infektion i.p.
b) KbE: Kolonien-bildende Einheiten

## 2. Wirkung auf Überlebensrate und Überlebenszeit

Die Gabe der erfindungsgemäßen Verbindungen führte auch zu einer Erhöhung der Überlebensrate und zu einem schnelleren Abklingen von Krankheitssymptomen.

Beispiele

Die Verbindungen Beispiel 13 g oder Beispiel 5 g wurden Mäusen subcutan einmalig vor letaler Infektion mit S. typhimurium verabreicht (Tabellen 2 und 3). Die Substanzen bewirkten in Dosen von 0,1; 1,0 oder 10 mg/kg eine Erhöhung der Überlebensrate und eine Verlängerung der Überlebenszeit. Ähnliche

Effekte konnten auch nach Infektion mit anderen Bakterien beobachtet werden. Bei den Tieren handelte es sich um weibliche Auszuchtmäuse des Stammes CF1 (Gewicht 18-20 g). Sie wurden in Makrolon-Käfigen unter konstanten Bedingungen (22 ± 2 °C; 55-65 % relative Luftfeuchtigkeit) gehalten und erhielten Ssniff Versuchstierdiät.

Tabelle 2

| Wirkung der Verbindung Beispiel 13 g auf die Überlebensrate und die Überlebenszeit von Mäusen nach letaler Infektion mit Salmonella typhimurium | | | |
|---|---|---|---|
| Dosis[a] (mg/kg) | Überlebensrate am 28. Tag nach der Infektion | p | [b] Überlebenszeit (Median) |
| Kontrolle | 1/24 ( 4 %) | | 7,5 Tage |
| 0,1 | 7/24 (29 %) | 0,02 | 13,0 Tage |
| 1,0 | 9/24 (38 %) | 0,005 | 10,0 Tage |
| 10,0 | 11/24 (46 %) | 0,0009 | 14,0 Tage |

a) einmalige subcutane Applikation 24 h vor der Infektion
b) im Vergleich zur Kontrolle, Fisher's exact test

Tabelle 3

| Wirkungen der Verbindung Beispiel 5 g auf die Überlebensrate und die Überlebenszeit von Mäusen nach letaler Infektion mit Salmonella typhimurium | | | |
|---|---|---|---|
| Dosis[a] (mg/kg) | Überlebensrate am 28. Tag nach der Infektion | p | [b] Überlebenszeit (Median) |
| Kontrolle | 0/24 | | 7,5 Tage |
| 0,1 | 3/24 (13 %) | 0,12 | 7,5 Tage |
| 1,0 | 6/24 (25 % | 0,01 | 8,0 Tage |
| 10,0 | 9/24 (38 %) | 0,0008 | 17,5 Tage |

a) einmalige subcutane Applikation 24 h vor der Infektion
b) im Vergleich zur Kontrolle, Fisher's exact test

Die erfindungsgemäßen Verbindungen der Formel I zeigen außerdem in vitro Wirkung auf Humangranulozyten.

Die Phagozyten sind wichtiger Bestandteil der unspezifischen Infektionsabwehr. Als erste zelluläre "Verteidigungslinie" des Immunsystems wehren sie eindringende Mikroorganismen ab und sind somit von ausschlaggebender Bedeutung für den weiteren Verlauf des Infektionsgeschehens.

Neben verschiedenen anderen mikrobiziden Eigenschaften sind Phagozyten insbesondere in der Lage, über den oxidativen Metabolismus toxische, reaktive Sauerstoffspezies ($O_2^-$, $H_2O_2$, -OH, $^1O_2$) mit antimikrobieller Wirkung zu produzieren.

In entsprechenden Studien wurde der Einfluß der erfindungsgemäßen Verbindungen der allgemeinen Formel (I) auf den oxidativen Metabolismus geprüft. Es konnte, hier am Beispiel der Verbindung Beispiel 5 d dargestellt, gezeigt werden, daß der oxidative Metabolismus, gemessen anhand der Listerien-induzierten Chemilumineszenz und $O_2$-Produktion von humanen Granulozyten aus peripherem Blut, deutlich erhöht wurde. Im Falle der Verbindung Beispiel 5 d wurde eine maximale Wirkung bei einer Konzentration von 10 $\mu g/10^6$ Zellen/ml erreicht (Abb. 1a und 1b).

Steigerung der primären humoralen Immunität in vitro gegen Schaferythrozyten (SE)

Es ist experimentell möglich, die Entwicklung einer humoralen Immunantwort mit heterologen roten

Blutzellen durch primäre Immunisation von Maus-Milzzellen in Suspensionskulturen in vitro einzuleiten (R.I. Mishell und R.W. Dutton, J. Exp. Med. 126, 423 (1967)). Hierzu werden Balb/c Maus-Milzzellen für 5 Tage in Gegenwart von Antigen (SE) und der Testsubstanz kultiviert. Die Zellen werden geerntet, gewaschen und zusammen mit dem Antigen und Komplement in semisolidem Agar ausplattiert und für 2 Stunden bei 37° C inkubiert (N.K. Jerne, A.A. Nordin und C. Henry, "Cell bound Antibodies", eds. Amos and Koprowski, Wistar Inst. Press, Philadelphia, USA, pp 109 (1963)). Die Antigen-Sensibilisierung von Maus-Lymphozyten in der Primärkultur resultiert in der Synthese und Freisetzung von Antikörpern. Die spezifischen, sezernierten Antikörper binden sich an das SE-Antigen und lysieren diese Zellen durch die Gegenwart des Komplements (Plaque-Bildung). Substanzen der vorliegenden Verbindungsklasse sind in der Lage, dosisabhängig im Bereich 3 - 100 µg/ml die Zahl der Antikörper-bildenden Zellen zu steigern (Tabelle 4).

| Wirkungen der Verbindung Beispiel 13 g auf die Antikörpersynthese in vitro . Antikörper sezernierende Zellen/Kultur in Abhängigkeit von der Dosis [µg/ml] | | | | | | |
|---|---|---|---|---|---|---|
| Dosis [µg/ml] | 0 | 0,1 | 0,3 | 1,0 | 3,0 | 10,0 |
| Antikörper sezernierende zellen | 1540 | 2100 | 2540 | 7150 | 13800 | 8560 |

Die erfindungsgemäßen Verbindungen können als pharmazeutische Präparate formuliert werden. Bevorzugte pharmazeutische Präparate sind Tabletten oder Gelatinekapseln, die die Wirkstoffe zusammen mit folgenden Verdünnungsmitteln enthalten: z.B. Lactose, Dextrose, Saccharose, Mannit, Sorbit, Cellulose und/oder Schmiermitteln, z.B. Kieselerde, Talk, Stearinsäure oder Salze davon, wie Magnesium- oder Calciumstearat, und/oder Polyethylen glykol. Tabletten enthalten ebenfalls Bindemittel, z.B. Magnesiumaluminiumsilikat, Stärken, wie Mais-, Weizen-, Reis- oder Pfeilwurzstärke, Gelatine, Traganth, Methylcellulose, Natriumcarboxymethylcellulose und/oder Polyvinylpyrrolidon, und wenn erwünscht, Sprengmittel, z.B. Stärken, Agar, Alginsäure oder ein Salz davon, wie Natriumalginat und/oder Brausemischungen, oder Adsorptionsmittel, Farbstoffe, Geschmackstoffe und Süßmittel. Injizierbare Präparate sind vorzugsweise isotonische wäßrige Lösungen oder Suspensionen. Suppositorien, Salben oder Cremen sind in erster Linie Fettemulsionen oder -suspensionen. Die pharmazeutischen Präparate der vorliegenden Erfindung können sterilisert sein und/oder Hilfsstoffe, z.B. Konservier-, Stabilisier-, Netz-und/oder Emulgiermittel, Löslichkeitsvermittler, Salze zur Regulierung des osmotischen Druckes und/oder Puffer enthalten. Die vorliegenden pharmazeutischen Präparate, die wenn erwünscht, weitere pharmakologisch wertvolle Stoffe enthalten können, werden in an sich bekannter Weise, z.B. mittels konventioneller Misch-, Granulier-oder Dragierverfahren, hergestellt und enthalten von etwa 0,1 % bis etwa 75 %, insbesondere von etwa 1 % bis 50 %, der genannten Aktivstoffe.

Die auch applizierten Präparate der vorliegenden Erfindung können auch mit einem gegen Magensaft beständigen Überzug versehen werden.

Die neuen Wirkstoffe können als abwehrsteigende und immunpotenzierende Mittel zur Behandlung von chronischen und akuten Infektionen (z.B. bakterielle, virale und parasitäre) und malignen Tumoren verwendet werden. Sie können ferner als Adjuvantien bei der Vakzinierung zur Stimulierung von Phagozytose, zur Modulierung des Abwehr- und Immunsystems verwendet werden.

Die erfindungsgemäßen Verbindungen und ihre pharmazeutisch verwendbaren, nicht-toxischen Salze zeichnen sich durch eine gute Löslichkeit in polaren Lösungsmitteln, vorzugsweise in Wasser, aus, wobei die Wirksamkeit überraschenderweise erhalten bleibt.

## Beispiele

Die Dünnschichtchromatographie (DC) erfolgte auf Kieselgel-DC-Fertigplatten (E. Merck, Darmstadt) und die präparative Trennungen mit Kieselgel 60 (Merck, Darmstadt).

### Allgemeine Vorschrift für die Umsetzung mit acyclischen Dicarbonsäuremonoalkylestern (Variante A)

1 mmol der Aminkomponente werden in 20 ml Ethanol gelöst und nach Zugabe von 1 mmol Dicarbonsäuremonoalkylester und 1 ml 1 N Natronlauge über Nacht gerührt. Die Reaktionsmischung wird im Vakuum eingedampft, in 5 ml Ethanol und 50 ml Wasser aufgenommen und lyophilisiert.

Allgemeine Vorschrift für die Umsetzung mit cyclischen Dicarbonsäureanhydriden (Variante B)

1 mmol der Aminkomponente werden in 20 ml Ethanol gelöst und nach Zugabe von 1 mmol Dicarbonsäureanhydrid über Nacht gerührt. Nach vollständiger Umsetzung wird mit 1 ml 1 N Natronlauge neutralisiert und im Vakuum eingedampft. Der Rückstand wird dann in 5 ml Ethanol und 50 ml Wasser aufgenommen und lyophilisiert.

Beispiel 1

Durch die Umsetzung von N-(2-Amino-2-desoxy-β-D-glucopyranosyl)-N-alkylcarbonsäureamiden mit Bernsteinsäureanhydrid erhält man die folgenden Verbindungen: (Variante B)

1.a N-[2-(3-Carboxypropionylamino)-2-desoxy-β-D-glucopyranosyl]-N-dodecyl-dodecanamid
1.b N-[2-(3-Carboxypropionylamino)-2-desoxy-β-D-glucopyranosyl]-N-dodecyl-tetradecanamid
1.c N-[2-(3-Carboxypropionylamino)-2-desoxy-β-D-glucopyranosyl]-N-dodecyl-octadecanamid
$[\alpha]_D$ = 11,8° (c = 0,7 in DMF)
$R_f$ = 0,13 in $CH_2Cl_2/CH_3OH/NH_3$-Wasser = 10:3:0,1 (v/v/v)
1.d N-[2-(3-Carboxypropionylamino)-2-desoxy-β-D-glucopyranosyl]-N-tetradecyl-dodecanamid
1.e N-[2-(3-Carboxypropionylamino)-2-desoxy-β-D-glucopyranosyl]-N-tetradecyl-tetradecanamid
1.f N-[2-(3-Carboxypropionylamino)-2-desoxy-β-D-glucopyranosyl]-N-tetradecyl-octadecanamid
1.g N-[2-(3-Carboxypropionylamino)-2-desoxy-β-D-glucopyranosyl]-N-octadecyl-dodecanamid
$[\alpha]_D$ = 12,0° (c = 0,5 in DMF)
$R_f$ = 0,15 in $CH_2Cl_2/CH_3OH/NH_3$-Wasser = 10:3:0,1 (v/v/v)
1.h N-[2-(3-Carboxypropionylamino)-2-desoxy-β-D-glucopyranosyl]-N-octadecyl-tetradecanamid
1.i N-[2-(3-Carboxypropionylamino)-2-desoxy-β-D-glucopyranosyl]-N-octadecyl-octadecanamid

Beispiel 2

Umsetzung von N-(2-Amino-2-desoxy-β-D-glucopyranosyl)-N-alkylcarbonsäureamiden mit Glutarsäureanhydrid (Variante B)

2.a N-[2-(4-Carboxybutyrylamino)-2-desoxy-β-D-glucopyranosyl]-N-dodecyl-octadecanamid
$[\alpha]_D$ = 11,6° (c = 0,7 in DMF)
2.b N-[2-(4-Carboxybutyrylamino)-2-desoxy-β-D-glucopyranosyl]-N-tetradecyl-octadecanamid
2.c N-[2-(4-Carboxybutyrylamino)-2-desoxy-β-D-glucopyranosyl]-N-octadecyl-dodecanamid
2.d N-[2-(4-Carboxybutyrylamino)-2-desoxy-β-D-glucopyranosyl]-N-octadecyl-octadecanamid

Beispiel 3

Umsetzung von N-(2-Amino-2-desoxy-β-D-glucopyranosyl)-N-alkylcarbonsäureamiden mit Adipinsäuremonomethylester (Variante A)

3.a N-[2-(5-Carboxyvalerylamino)-2-desoxy-β-D-glucopyranosyl]-N-dodecyl-octadecanamid
3.b N-[2-(5-Carboxyvalerylamino)-2-desoxy-β-D-glucopyranosyl]-N-tetradecyl-octadecanamid
3.c N-[2-(5-Carboxyvalerylamino)-2-desoxy-β-D-glucopyranosyl]-N-octadecyl-dodecanamid
3.d N-[2-(5-Carboxyvalerylamino)-2-desoxy-β-D-glucopyranosyl]-N-octadecyl-octadecanamid

Beispiel 4

Umsetzung von N-(2-Amino-2-desoxy-β-D-glucopyranosyl)-N-alkylcarbonsäureamiden mit Octandicarbonsäuremonomethylester (Variante A)

4.a N-[2-(7-Carboxyheptanoylamino)-2-desoxy-β-D-glucopyranosyl]-N-dodecyl-octadecanamid
$[\alpha]_D$ = 10,8° (c = 0,5 in DMF)
4.b N-[2-(7-Carboxyheptanoylamino)-2-desoxy-β-D-glucopyranosyl]-N-tetradecyl-octadecanamid

4.c N-[2-(7-Carboxyheptanoylamino)-2-desoxy-β-D-glucopyranosyl]-N-octadecyl-dodecanamid
4.d N-[2-(7-Carboxyheptanoylamino)-2-desoxy-β-D-glucopyranosyl]-N-octadecyl-octadecanamid

Beispiel 5

Umsetzung von N-(2-Glycylamino-2-desoxy-β-D-glucopyranosyl)-N-alkyl-carbonsäureamiden mit Bernsteinsäureanhydrid (Variante B)

5.a N-{2-[N-(3-Carboxypropionyl)-glycyl]-amino-2-desoxy-β-D-glucopyranosyl}-N-dodecyl-dodecanamid

5.b N-{2-[N-(3-Carboxypropionyl)-glycyl]-amino-2-desoxy-β-D-glucopyranosyl}-N-dodecyl-tetradecanamid

5.c N-{2-[N-(3-Carboxypropionyl)-glycyl]-amino-2-desoxy-β-D-glucopyranosyl}-N-dodecyl-octadecanamid

5.d N-{2-[N-(3-Carboxypropionyl)-glycyl]-amino-2-desoxy-β-D-glucopyranosyl}-N-tetradecyl-dodecanamid
$[\alpha]_D$ = 4,9° (c = 1,4 in Methanol)
$R_f$ = 0,15 in $CH_2Cl_2/CH_3OH/NH_3$-Wasser = 10:3:0,1 (v/v/v)

5.e N-{2-[N-(3-Carboxypropionyl)-glycyl]-amino-2-desoxy-β-D-glucopyranosyl}-N-tetradecyl-tetradecanamid

5.f N-{2-[N-(3-Carboxypropionyl)-glycyl]-amino-2-desoxy-β-D-glucopyranosyl}-N-tetradecyl-octadecanamid

5.g N-{2-[N-(3-Carboxypropionyl)-glycyl]-amino-2-desoxy-β-D-glucopyranosyl}-N-octadecyl-dodecanamid
$[\alpha]_D$ = 8,8° (c = 0,5 in DMF)
$R_f$ = 0,10 in $CH_2Cl_2/CH_3OH/NH_3$-Wasser = 10:3:0,1 (v/v/v)

5.h N-{2-[N-(3-Carboxypropionyl)-glycyl]-amino-2-desoxy-β-D-glucopyranosyl}-N-octadecyl-tetradecanamid

5.i N-{2-[N-(3-Carboxypropionyl)-glycyl]-amino-2-desoxy-β-D-glucopyranosyl}-N-octadecyl-octadecanamid

Beispiel 6

Umsetzung von N-(2-Glycylamino-2-desoxy-β-D-glucopyranosyl)-N-alkyl-carbonsäureamiden mit Glutarsäureanhydrid (Variante B)

6.a N-{2-[N-(4-Carboxybutyryl)-glycyl]-amino-2-desoxy-β-D-glucopyranosyl}-N-dodecyl-octadecanamid

6.b N-{2-[N-(4-Carboxybutyryl)-glycyl]-amino-2-desoxy-β-D-glucopyranosyl}-N-tetradecyl-octadecanamid

6.c N-{2-[N-(4-Carboxybutyryl)-glycyl]-amino-2-desoxy-β-D-glucopyranosyl}-N-octadecyl-dodecanamid

6.d N-{2-[N-(4-Carboxybutyryl)-glycyl]-amino-2-desoxy-β-D-glucopyranosyl}-N-octadecyl-octadecanamid

Beispiel 7

Umsetzung von N-(2-Glycylamino-2-desoxy-β-D-glucopyranosyl)-N-alkyl-carbonsäureamiden mit Adipinsäuremonomethylester (Variante A)

7.a N-{2-[N-(5-Carboxyvaleryl)-glycyl]-amino-2-desoxy-β-D-glucopyranosyl}-N-dodecyl-octadecanamid

7.b N-{2-[N-(5-Carboxyvaleryl)-glycyl]-amino-2-desoxy-β-D-glucopyranosyl}-N-tetradecyl-octadecanamid

7.c N-{2-[N-(5-Carboxyvaleryl)-glycyl]-amino-2-desoxy-β-D-glucopyranosyl}-N-octadecyl-dodecanamid

7.d N-{2-[N-(5-Carboxyvaleryl)-glycyl]-amino-2-desoxy-β-D-glucopyranosyl}-N-octadecyl-octadecanamid

Beispiel 8

Umsetzung von N-(2-Glycylamino-2-desoxy-β-D-glucopyranosyl)-N-alkyl-carbonsäureamiden mit Octandicarbonsäuremonomethylester (Variante A)

8.a N-{2-[N-(7-Carboxyheptanoyl)-glycyl]-amino-2-desoxy-β-D-glucopyranosyl}-N-dodecyl-octadecanamid

8.b N-{2-[N-(7-Carboxyheptanoyl)-glycyl]-amino-2-desoxy-β-D-glucopyranosyl}-N-tetradecyl-octadecanamid

8.c N-{2-[N-(7-Carboxyheptanoyl)-glycyl]-amino-2-desoxy-β-D-glucopyranosyl}-N-octadecyl-dodecanamid

8.d N-{2-[N-(7-Carboxyheptanoyl)-glycyl]-amino-2-desoxy-β-D-glucopyranosyl}-N-octadecyl-octadecanamid

Beispiel 9

Umsetzung von N-(2-L-Alanylamino-2-desoxy-β-D-glucopyranosyl)-N-alkyl-carbonsäureamiden mit Bernsteinsäureanhydrid (Variante B)

9.a N-{2-[N-(3-Carboxypropionyl)-L-alanyl]-amino-2-desoxy-β-D-glucopyranosyl}-N-dodecyl-octadecanamid

9.b N-{2-[N-(3-Carboxypropionyl)-L-alanyl]-amino-2-desoxy-β-D-glucopyranosyl}-N-tetradecyl-octadecanamid

9.c N-{2-[N-(3-Carboxypropionyl)-L-alanyl]-amino-2-desoxy-β-D-glucopyranosyl}-N-octadecyl-dodecanamid

.a N-{2-[N-(3-Carboxypropionyl)-L-alanyl]-amino-2-desoxy-β-D-glucopyranosyl}-N-octadecyl-octadecanamid

Beispiel 10

Umsetzung von N-(2-L-Alanylamino-2-desoxy-β-D-glucopyranosyl)-N-alkyl-carbonsäureamiden mit Glutarsäureanhydrid (Variante B)

10.a N-{2-[N-(4-Carboxybutyryl)-L-alanyl]-amino-2-desoxy-β-D-glucopyranosyl}-N-dodecyl-octadecanamid

10.b N-{2-[N-(4-Carboxybutyryl)-L-alanyl]-amino-2-desoxy-β-D-glucopyranosyl}-N-tetradecyl-octadecanamid

10.c N-{2-[N-(4-Carboxybutyryl)-L-alanyl]-amino-2-desoxy-β-D-glucopyranosyl}-N-octadecyl-dodecanamid

10.d N-{2-[N-(4-Carboxybutyryl)-L-alanyl]-amino-2-desoxy-β-D-glucopyranosyl}-N-octadecyl-octadecanamid

Beispiel 11

Umsetzung von N-(2-L-Alanylamino-2-desoxy-β-D-glucopyranosyl)-N-alkyl-carbonsäureamiden mit Adipinsäuremonomethylester (Variante A)

11.a N-{2-[N-(5-Carboxyvaleryl)-L-alanyl]-amino-2-desoxy-β-D-glucopyranosyl}-N-dodecyl-octadecanamid

11.b N-{2-[N-(5-Carboxyvaleryl)-L-alanyl]-amino-2-desoxy-β-D-glucopyranosyl}-N-tetradecyl-octadecanamid

11.c N-{2-[N-(5-Carboxyvaleryl)-L-alanyl]-amino-2-desoxy-β-D-glucopyranosyl}-N-octadecyl-dodecanamid

11.d N-{2-[N-(5-Carboxyvaleryl)-L-alanyl]-amino-2-desoxy-β-D-glucopyranosyl}-N-octadecyl-octadecanamid

## Beispiel 12

Umsetzung von N-(2-L-Alanylamino-2-desoxy-β-D-glucopyranosyl)-N-alkyl-carbonsäureamiden mit Octandicarbonsäuremonomethylester (Variante A)

12.a N-{2-[N-(8-Carboxyheptanoyl)-L-alanyl]-amino-2-desoxy-β-D-glucopyranosyl}-N-dodecyl-octadecanamid

12.b N-{2-[N-(8-Carboxyheptanoyl)-L-alanyl]-amino-2-desoxy-β-D-glucopyranosyl}-N-tetradecyl-octadecanamid

12.c N-{2-[N-(8-Carboxyheptanoyl)-L-alanyl]-amino-2-desoxy-β-D-glucopyranosyl}-N-octadecyl-dodecanamid

12.d N-{2-[N-(8-Carboxyheptanoyl)-L-alanyl]-amino-2-desoxy-β-D-glucopyranosyl}-N-octadecyl-octadecanamid

## Beispiel 13

Umsetzung von N-(2-L-Leucylamino-2-desoxy-β-D-glucopyranosyl)-N-alkyl-carbonsäureamiden mit Bernsteinsäureanhydrid (Variante B)

13.a N-{2-[N-(3-Carboxypropionyl)-L-leucyl]-amino-2-desoxy-β-D-glucopyranosyl}-N-dodecyl-dodecanamid

13.b N-{2-[N-(3-Carboxypropionyl)-L-leucyl]-amino-2-desoxy-β-D-glucopyranosyl}-N-dodecyl-tetradecanamid

13.c N-{2-[N-(3-Carboxypropionyl)-L-leucyl]-amino-2-desoxy-β-D-glucopyranosyl}-N-dodecyl-octadecanamid

$R_f$ = 0,13 in $CH_2Cl_2/CH_3OH/NH_3$-Wasser = 10:3:0,1 (v/v/v)

13.d N-{2-[N-(3-Carboxypropionyl)-L-leucyl]-amino-2-desoxy-β-D-glucopyranosyl}-N-tetradecyl-dodecanamid

13.e N-{2-[N-(3-Carboxypropionyl)-L-leucyl]-amino-2-desoxy-β-D-glucopyranosyl}-N-tetradecyl-tetradecanmid

13.f N-{2-[N-(3-Carboxypropionyl)-L-leucyl]-amino-2-desoxy-β-D-glucopyranosyl}-N-tetradecyl-octadecanamid

13.g N-{2-[N-(3-Carboxypropionyl)-L-leucyl]-amino-2-desoxy-β-D-glucopyranosyl}-N-octadecyl-dodecanamid

$R_f$ = 0,15 in $CH_2Cl_2/CH_3OH/NH_3$-Wasser = 10:3:0,1 (v/v/v)

13.h N-{2-[N-(3-Carboxypropionyl)-L-leucyl]-amino-2-desoxy-β-D-glucopyranosyl}-N-octadecyl-tetradecanamid

13.i N-{2-[N-(3-Carboxypropionyl)-L-leucyl]-amino-2-desoxy-β-D-glucopyranosyl}-N-octadecyl-octadecanamid

## Beispiel 14

Umsetzung von N-(2-L-Leucylamino-2-desoxy-β-D-glucopyranosyl)-N-alkyl-carbonsäureamiden mit Glutarsäureanhydrid (Variante B)

14.a N-{2-[N-(4-carboxybutyryl)-L-leucyl]-amino-2-desoxy-β-D-glucopyranosyl}-N-dodecyl-octadecanamid

14.b N-{2-[N-(4-carboxybutyryl)-L-leucyl]-amino-2-desoxy-β-D-glucopyranosyl}-N-tetradecyl-octadecanamid

14.c N-{2-[N-(4-carboxybutyryl)-L-leucyl]-amino-2-desoxy-β-D-glucopyranosyl}-N-octadecyl-dodecanamid

14.d N-{2-[N-(4-carboxybutyryl)-L-leucyl]-amino-2-desoxy-β-D-glucopyranosyl}-N-octadecyl-octadecanamid

Beispiel 15

Umsetzung von N-(2-L-Leucylamino-2-desoxy-β-D-glucopyranosyl)-N-alkyl-carbonsäureamiden mit Adipinsäuremonomethylester (Variante A)

15.a N-{2-[N-(5-Carboxyvaleryl)-L-leucyl]-amino-2-desoxy-β-D-glucopyranosyl}-N-dodecyl-octadecanamid

15.b N-{2-[N-(5-Carboxyvaleryl)-L-leucyl]-amino-2-desoxy-β-D-glucopyranosyl}-N-tetradecyl-octadecanamid

15.c N-{2-[N-(5-Carboxyvaleryl)-L-leucyl]-amino-2-desoxy-β-D-glucopyranosyl}-N-octadecyl-dodecanamid

15.d N-{2-[N-(5-Carboxyvalelryl)-L-leucyl]-amino-2-desoxy-β-D-glucopyranosyl}-N-octadecyl-octadecanamid

Beispiel 16

Umsetzung von N-(2-L-Leucylamino-2-desoxy-β-D-glucopyranosyl)-N-alkyl-carbonsäureamiden mit Octandicarbonsäuremonomethylester (Variante A)

16.a N-{2-[N-(7-Carboxyheptanoyl)-L-leucyl]-amino-2-desoxy-β-D-glucopyranosyl}-N-dodecyl-octadecanamid

16.b N-{2-[N-(7-Carboxyheptanoyl)-L-leucyl]-amino-2-desoxy-β-D-glucopyranosyl}-N-tetradecyl-octadecanamid

16.c N-{2-[N-(7-Carboxyheptanoyl)-L-leucyl]-amino-2-desoxy-β-D-glucopyranosyl}-N-octadecyl-dodecanamid

16.d N-{2-[N-(7-Carboxyheptanoyl)-L-leucyl]-amino-2-desoxy-β-D-glucopyranosyl}-N-octadecyl-octadecanamid

Beispiel 17

Umsetzung von N-(2-L-Serylamino-2-desoxy-β-D-glucopyranosyl)-N-alkyl-carbonsäureamiden mit Bernsteinsäureanhydrid (Variante B)

17.a N-{2-[N-(3-Carboxypropionyl)-L-seryl]-amino-2-desoxy-β-D-glucopyranosyl}-N-dodecyl-octadecanamid

17.b N-{2-[N-(3-Carboxypropionyl)-L-seryl]-amino-2-desoxy-β-D-glucopyranosyl}-N-tetradecyl-octadecanamid

17.c N-{2-[N-(3-Carboxypropionyl)-L-seryl]-amino-2-desoxy-β-D-glucopyranosyl}-N-octadecyl-dodecanamid

17.d N-{2-[N-(3-Carboxypropionyl)-L-seryl]-amino-2-desoxy-β-D-glucopyranosyl}-N-octadecyl-octadecanamid

Beispiel 18

Umsetzung von N-(2-L-Serylamino-2-desoxy-β-D-glucopyranosyl)-N-alkyl-carbonsäureamiden mit Glutarsäureanhydrid (Variante B)

18.a N-{2-[N-(4-Carboxybutyryl)-L-seryl]-amino-2-desoxy-β-D-glucopyranosyl}-N-dodecyl-octadecanamid

18.b N-{2-[N-(4-Carboxybutyryl)-L-seryl]-amino-2-desoxy-β-D-glucopyranosyl}-N-tetradecyl-octadecanamid

18.c N-{2-[N-(4-Carboxybutyryl)-L-seryl]-amino-2-desoxy-β-D-glucopyranosyl}-N-octadecyl-dodecanamid

18.d N-{2-[N-(4-Carboxybutyryl)-L-seryl]-amino-2-desoxy-β-D-glucopyranosyl}-N-octadecyl-octadecanamid

Beispiel 19

Umsetzung von N-(2-L-Serylamino-2-desoxy-β-D-glucopyranosyl)-N-alkyl-carbonsäureamiden mit Adipinsäuremonomethylester (Variante A)

19.a N-{2-[N-5-Carboxyvaleryl)-L-seryl]-amino-2-desoxy-β-D-glucopyranosyl}-N-dodecyl-octadecanamid

19.b N-{2-[N-5-Carboxyvaleryl)-L-seryl]-amino-2-desoxy-β-D-glucopyranosyl}-N-tetradecyl-octadecanamid

19.c. N-{2-[N-5-Carboxyvaleryl)-L-seryl]-amino-2-desoxy-β-D-glucopyranosyl}-N-octadecyl-dodecansäureamid

19.d N-{2-[N-5-Carboxyvaleryl)-L-seryl]-amino-2-desoxy-β-D-glucopyranosyl}-N-octadecyl-octadecanamid

Beispiel 20

Umsetzung von N-(2-L-Serylamino-2-desoxy-β-D-glucopyranosyl)-N-alkyl-carbonsäureamiden mit Octandicarbonsäuremonomethylester (Variante A)

20.a N-{2-[N-(7-Carboxyheptanoyl)-L-seryl]-amino-2-desoxy-β-D-glucopyranosyl}-N-dodecyl-octadecanamid

20.b N-{2-[N-(7-Carboxyheptanoyl)-L-seryl]-amino-2-desoxy-β-D-glucopyranosyl}-N-tetradecyl-octadecanamid

20.c N-{2-[N-(7-Carboxyheptanoyl)-L-seryl]-amino-2-desoxy-β-D-glucopyranosyl}-N-octadecyl-dodecanamid

20.d N-{2-[N-(7-Carboxyheptanoyl)-L-seryl]-amino-2-desoxy-β-D-glucopyranosyl}-N-octadecyl-octadecanamid

Beispiel 21

Umsetzung von N-(2-L-Phenylalanylamino-2-desoxy-β-D-glucopyranosyl)-N-alkyl-carbonsäureamiden mit Bernsteinsäureanhydrid (Variante B)

21.a N-{2-[N-(3-Carboxypropionyl)-L-phenylalanyl]-amino-2-desoxy-β-D-glucopyranosyl}-N-dodecyl-octadecanamid

21.b N-{2-[N-(3-Carboxypropionyl)-L-phenylalanyl]-amino-2-desoxy-β-D-glucopyranosyl}-N-tetradecyl-octadecanamid

21.c N-{2-[N-(3-Carboxypropionyl)-L-phenylalanyl]-amino-2-desoxy-β-D-glucopyranosyl}-N-octadecyl-dodecanamid

21.d N-{2-[N-(3-Carboxypropionyl)-L-phenylalanyl]-amino-2-desoxy-β-D-glucopyranosyl}-N-octadecyl-octadecanamid

Beispiel 22

Umsetzung von N-(2-L-Phenylalanylamino-2-desoxy-β-D-glucopyranosyl)-N-alkyl-carbonsäureamiden mit Glutarsäureanhydrid (Variante B)

22.a N-{2-[N-(4-Carboxybutyryl)-L-phenylalanyl]-amino-2-desoxy-β-D-glucopyranosyl}-N-dodecyl-octadecanamid

22.b N-{2-[N-(4-Carboxybutyryl)-L-phenylalanyl]-amino-2-desoxy-β-D-glucopyranosyl}-N-tetradecyl-octadecanamid

22.c N-{2-[N-(4-Carboxybutyryl)-L-phenylalanyl]-amino-2-desoxy-β-D-glucopyranosyl}-N-octadecyl-dodecanamid

22.d N-{2-[N-(4-Carboxybutyryl)-L-phenylalanyl]-amino-2-desoxy-β-D-glucopyranosyl}-N-octadecyl-octadecanamid

Beispiel 23

Umsetzung von N-(2-L-Phenylalanylamino-2-desoxy-β-D-glucopyranosyl)-N-alkyl-carbonsäureamiden mit Adipinsäuremonomethylester (Variante A)

23.a N-{2-[N-(5-Carboxyvaleryl)-L-phenylalanyl]-amino-2-desoxy-β-D-glucopyranosyl]-N-dodecyl-octadecanamid

23.b N-{2-[N-(5-Carboxyvaleryl)-L-phenylalanyl]-amino-2-desoxy-β-D-glucopyranosyl]-N-tetradecyl-octadecanamid

23.c N-{2-[N-(5-Carboxyvaleryl)-L-phenylalanyl]-amino-2-desoxy-β-D-glucopyranosyl]-N-octadecyl-dodecanamid

23.d N-{2-[N-(5-Carboxyvaleryl)-L-phenylalanyl]-amino-2-desoxy-β-D-glucopyranosyl]-N-octadecyl-octadecanamid

Beispiel 24

Umsetzung von N-(2-L-Phenylalanylamino-2-desoxy-β-D-glucopyranosyl)-N-alkyl-carbonsäureamiden mit Octandicarbonsäuremonomethylester (Variante A)

24.a N-{2-[N-(7-Carboxyheptanoyl)-L-phenylalanyl]-amino-2-desoxy-β-D-glucopyranosyl}-N-dodecyl-octadecanamid

24.b N-{2-[N-(7-Carboxyheptanoyl)-L-phenylalanyl]-amino-2-desoxy-β-D-glucopyranosyl}-N-tetradecyl-octadecanamid

24.c N-{2-[N-(7-Carboxyheptanoyl)-L-phenylalanyl]-amino-2-desoxy-β-D-glucopyranosyl}-N-octadecyl-dodecanamid

24.d N-{2-[N-(7-Carboxyheptanoyl)-L-phenylalanyl]-amino-2-desoxy-β-D-glucopyranosyl}-N-octadecyl-octadecanamid

Beispiel 25

Umsetzung von N-(2-Amino-2-desoxy-β-D-galactopyranosyl)-N-alkyl-carbonsäureamiden mit Bernsteinsäureanhydrid (Variante B)

25.a N-[2-(3-Carboxypropionylamino)-2-desoxy-β-D-galactopyranosyl]-N-dodecyl-dodecanamid
25.b N-[2-(3-Carboxypropionylamino)-2-desoxy-β-D-galactopyranosyl]-N-dodecyl-tetradecanamid
25.c N-[2-(3-Carboxypropionylamino)-2-desoxy-β-D-galactopyranosyl]-N-dodecyl-octadecanamid
25.d N-[2-(3-Carboxypropionylamino)-2-desoxy-β-D-galactopyranosyl]-N-tetradecyl-dodecanamid
25.e N-[2-(3-Carboxypropionylamino)-2-desoxy-β-D-galactopyranosyl]-N-tetradecyl-tetradecanamid
25.f N-[2-(3-Carboxypropionylamino)-2-desoxy-β-D-galactopyranosyl]-N-tetradecyl-octadecanamid
25.g N-[2-(3-Carboxypropionylamino)-2-desoxy-β-D-galactopyranosyl]-N-octadecyl-dodecanamid
25.h N-[2-(3-Carboxypropionylamino)-2-desoxy-β-D-galactopyranosyl]-N-octadecyl-tetradecanamid

25.i N-[2-(3-Carboxypropionylamino)-2-desoxy-β-D-galactopyranosyl]-N-octadecyl-octadecanamid

Beispiel 26

Umsetzung von N-(2-Amino-2-desoxy-β-D-galactopyranosyl)-N-alkyl-carbonsäureamiden mit Glutarsäureanhydrid (Variante B)

26.a N-[2-(4-Carboxybutyrylamino)-2-desoxy-β-D-galactopyranosyl]-N-dodecyl-octadecanamid
26.b N-[2-(4-Carboxybutyrylamino)-2-desoxy-β-D-galactopyranosyl]-N-tetradecyl-octadecanamid
26.c N-[2-(4-Carboxybutyrylamino)-2-desoxy-β-D-galactopyranosyl]-N-octadecyl-dodecanamid
26.d N-[2-(4-Carboxybutyrylamino)-2-desoxy-β-D-galactopyranosyl]-N-octadecyl-octadecanamid

Beispiel 27

Umsetzung von N-(2-Amino-2-desoxy-β-D-galactopyranosyl)-N-alkyl-carbonsäureamiden mit Adipinsäuremonomethylester (Variante A)

27.a N-[2-5-Carboxyvalerylamino)-2-desoxy-β-D-galactopyranosyl]-N-dodecyl-octadecanamid
27.b N-[2-5-Carboxyvalerylamino)-2-desoxy-β-D-galactopyranosyl]-N-tetradecyl-octadecanamid
27.c N-[2-5-Carboxyvalerylamino)-2-desoxy-β-D-galactopyranosyl]-N-octadecyl-dodecanamid
27.d N-[2-5-Carboxyvalerylamino)-2-desoxy-β-D-galactopyranosyl]-N-octadecyl-octadecanamid

Beispiel 28

Umsetzung von N-(2-Amino-2-desoxy-β-D-galactopyranosyl)-N-alkyl-carbonsäureamiden mit Octandicarbonsäuremonomethylester (Variante A)

28.a N-[2-(7-Carboxyheptanoylamino)-2-desoxy-β-D-galactopyranosyl]-N-dodecyl-octadecanamid
28.b N-[2-(7-Carboxyheptanoylamino)-2-desoxy-β-D-galactopyranosyl]-N-tetradecyl-octadecanamid
28.c N-[2-(7-Carboxyheptanoylamino)-2-desoxy-β-D-galactopyranosyl]-N-octadecyl-dodecanamid
28.d N-[2-(7-Carboxyheptanoylamino)-2-desoxy-β-D-galactopyranosyl]-N-octadecyl-octadecanamid

Beispiel 29

Umsetzung von N-(2-Glycylamino-2-desoxy-β-D-galactopyranosyl)-N-alkyl-carbonsäureamiden mit Bernsteinsäureanhydrid (Variante B)

29.a N-{2-[N-(3-Carboxypropionyl)-glycyl]-amino-2-desoxy-β-D-galactopyranosyl}-N-dodecyl-dodecanamid
29.b N-{2-[N-(3-Carboxypropionyl)-glycyl]-amino-2-desoxy-β-D-galactopyranosyl}-N-dodecyl-tetradecanamid
29.c N-{2-[N-(3-Carboxypropionyl)-glycyl]-amino-2-desoxy-β-D-galactopyranosyl}-N-dodecyl-octadecanamid
29.d N-{2-[N-(3-Carboxypropionyl)-glycyl]-amino-2-desoxy-β-D-galactopyranosyl}-N-tetradecyl-dodecanamid
29.e N-{2-[N-(3-Carboxypropionyl)-glycyl]-amino-2-desoxy-β-D-galactopyranosyl}-N-tetradecyl-tetradecanamid
29.f N-{2-[N-(3-Carboxypropionyl)-glycyl]-amino-2-desoxy-β-D-galactopyranosyl}-N-tetradecyl-octadecanamid
29.g N-{2-[N-(3-Carboxypropionyl)-glycyl]-amino-2-desoxy-β-D-galactopyranosyl}-N-octadecyl-dodecanamid
29.h N-{2-[N-(3-Carboxypropionyl)-glycyl]-amino-2-desoxy-β-D-galactopyranosyl}-N-octadecyl-tetradecanamid

29.i N-{2-[N-(3-Carboxypropionyl)-glycyl]-amino-2-desoxy-β-D-galactopyranosyl}-N-octadecyl-octadecanamid.

## Ansprüche

1. Verbindungen der allgemeinen Formel I

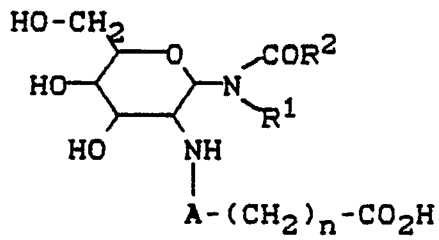

in der
$R^1$ einen gesättigten Alkylrest oder einen einfach, zweifach oder dreifach ungesättigten Alkenylrest mit bis zu 50 Kohlenstoffatomen bedeutet,
$R^2$ einen gesättigten Alkylrest oder einen einfach, zweifach oder dreifach ungesättigten Alkenylrest mit bis zu 50 Kohlenstoffatomen bedeutet,
A für -CO- oder für eine Gruppe

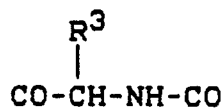

steht,
worin
$R^3$ für Wasserstoff, $C_1$-$C_7$-Alkyl, Hydroxymethyl, 1-Hydroxy-ethyl, Mercaptomethyl, 2-Methylthioethyl, 3-Aminopropyl, 3-Ureidopropyl, 3-Guanidylpropyl, 4-Aminobutyl, Carboxymethyl, Carbamoylmethyl, 2-Carboxyethyl, 2-Carbamoyl ethyl, Benzyl, 4-Hydroxybenzyl, 3-Indolylmethyl oder 4-Imidazolylmethyl steht und
n für 0 bis 10 steht und deren pharmazeutisch verwendbaren Salze.

2. Verbindungen gemäß Anspruch 1, worin $R^1$ und $R^2$ für Reste mit bis zu 30 Kohlenstoffatomen stehen.

3. Verbindungen gemäß Anspruch 2, worin $R^1$ und $R^2$ für Reste mit 10 bis 20 Kohlenstoffatomen stehen.

4. Verbindungen gemäß Ansprüchen 1 bis 3 in denen A für die Gruppe

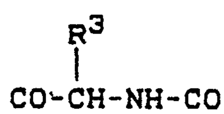

steht, worin $R^3$ für Wasserstoff, $C_1$-$C_7$-Alkyl, Hydroxymethyl, 1-Hydroxyethyl, Carboxymethyl, 2-Carboxyethyl, Benzyl oder 4-Hydroxybenzyl steht.

5. Verbindungen gemäß den Ansprüchen 1 bis 3 in den A für die D- oder L-Isomere der Aminosäuren wie Glycin, Sarcosin, Hippursäure, Alanin, Valin, Leucin, Isoleucin, Serin, Threonin, Cystein, Methionin, Ornithin, Citrullin, Arginin, Asparginsäure, Asparagin, Glutaminsäure, Glutamin, Phenylalanin, Tyrosin, Prolin, Tryptophan, Histidin oder für eine Aminocarbonsäure wie α-Aminobuttersäure oder α-Aminoheptansäure steht.

6. Verbindungen ausgewählt aus der Gruppe
1.a N-[2-(3-Carboxypropionylamino)-2-desoxy-β-D-glucopyranosyl]-N-dodecyl-dodecanamid,
1.b N-[2-(3-Carboxypropionylamino)-2-desoxy-β-D-glucopyranosyl]-N-dodecyl-tetradecanamid,
1.c N-[2-(3-Carboxypropionylamino)-2-desoxy-β-D-glucopyranosyl]-N-dodecyl-octadecanamid,
1.d N-[2-(3-Carboxypropionylamino)-2-desoxy-β-D-glucopyranosyl]-N-tetradecyl-dodecanamid,

1.e N-[2-(3-Carboxypropionylamino)-2-desoxy-β-D-glucopyranosyl]-N-tetradecyl-tetradecanamid,
1.f N-[2-(3-Carboxypropionylamino)-2-desoxy-β-D-glucopyranosyl]-N-tetradecyl-octadecanamid,
1.g N-[2-(3-Carboxypropionylamino)-2-desoxy-β-D-glucopyranosyl]-N-octadecyl-dodecanamid,
1.h N-[2-(3-Carboxypropionylamino)-2-desoxy-β-D-glucopyranosyl]-N-octadecyl-tetradecanamid,
1.i N-[2-(3-Carboxypropionylamino)-2-desoxy-β-D-glucopyranosyl]-N-octadecyl-octadecanamid,
2.a N-[2-(4-Carboxybutyrylamino)-2-desoxy-β-D-glucopyranosyl]-N-octadecyl-octadecanamid,
2b. N-[2-(4-Carboxybutyrylamino)-2-desoxy-β-D-glucopyranosyl]-N-tetradecyl-octadecanamid,
2c. N-[2-(4-Carboxybutyrylamino)-2-desoxy-β-D-glucopyranosyl]-N-octadecyl-dodecanamid,
2d. N-[2-(4-Carboxybutyrylamino)-2-desoxy-β-D-glucopyranosyl]-N-octadecyl-octadecanamid,
3.a N-[2-(5-Carboxyvalerylamino)-2-desoxy-β-D-glucopyranosyl]-N-dodecyl-octadecanamid,
3b. N-[2-(5-Carboxyvalerylamino)-2-desoxy-β-D-glucopyranosyl]-N-tetradecyl-octadecanamid,
3.c N-[2-(5-Carboxyvalerylamino)-2-desoxy-β-D-glucopyranosyl]-N-octadecyl-dodecanamid,
3.d N-[2-(5-Carboxyvalerylamino)-2-desoxy-β-D-glucopyranosyl]-N-octadecyl-octadecanamid,
4.a N-[2-(7-Carboxyheptanoylamino)-2-desoxy-β-D-glucopyranosyl]-N-dodecyl-octadecanamid,
4.b N-[2-(7-Carboxyheptanoylamino)-2-desoxy-β-D-glucopyranosyl]-N-tetradecyl-octadecanamid,
4.c N-[2-(7-Carboxyheptanoylamino)-2-desoxy-β-D-glucopyranosyl]-N-octadecyl-dodecanamid,
4.d N-[2-(7-Carboxyheptanoylamino)-2-desoxy-β-D-glucopyranosyl]-N-octadecyl-octadecanamid,
5.a N-{2-[N-(3-Carboxypropionyl)-glycyl]-amino-2-desoxy-β-D-glucopyranosyl}-N-dodecyl-dodecanamid,
5.b N-{2-[N-(3-Carboxypropionyl)-glycyl]-amino-2-desoxy-β-D-glucopyranosyl}-N-dodecyl-tetradecanamid,
5.c N-{2-[N-(3-Carboxypropionyl)-glycyl]-amino-2-desoxy-β-D-glucopyranosyl}-N-dodecyl-octadecanamid,
5.d N-{2-[N-(3-Carboxypropionyl)-glycyl]-amino-2-desoxy-β-D-glucopyranosyl}-N-tetradecyl-dodecanamid,
5.e N-{2-[N-(3-Carboxypropionyl)-glycyl]-amino-2-desoxy-β-D-glucopyranosyl}-N-tetradecyl-tetradecanamid,
5.f N-{2-[N-(3-Carboxypropionyl)-glycyl]-amino-2-desoxy-β-D-glucopyranosyl}-N-tetradecyl-octadecanamid,
5.g N-{2-[N-(3-Carboxypropionyl)-glycyl]-amino-2-desoxy-β-D-glucopyranosyl}-N-octadecyl-dodecanamid,
5.h N-{2-[N-(3-Carboxypropionyl)-glycyl]-amino-2-desoxy-β-D-glucopyranosyl}-N-octadecyl-tetradecanamid,
5.i N-{2-[N-(3-Carboxypropionyl)-glycyl]-amino-2-desoxy-β-D-glucopyranosyl}-N-octadecyl-octadecanamid,
6.a N-{2-[N-(4-Carboxybutyryl)-glycyl]-amino-2-desoxy-β-D-glucopyranosyl}-N-dodecyl-octadecanamid,
6.b N-{2-[N-(4-Carboxybutyryl)-glycyl]-amino-2-desoxy-β-D-glucopyranosyl}-N-tetradecyl-octadecanamid,
6.c N-{2-[N-(4-Carboxybutyryl)-glycyl]-amino-2-desoxy-β-D-glucopyranosyl}-N-octadecyl-dodecanamid,
6.d N-{2-[N-(4-Carboxybutyryl)-glycyl]-amino-2-desoxy-β-D-glucopyranosyl}-N-octadecyl-octadecanamid,
7.a N-{2-[N-(5-Carboxyvaleryl)-glycyl]-amino-2-desox-β-D-glucopyranosyl}-N-dodecyl-octadecanamid,
7.b N-{2-[N-(5-Carboxyvaleryl)-glycyl]-amino-2-desox-β-D-glucopyranosyl}-N-tetradecyl-octadecanamid,
7.c N-{2-[N-(5-Carboxyvaleryl)-glycyl]-amino-2-desox-β-D-glucopyranosyl}-N-octadecyl-dodecanamid,
7.d N-{2-[N-(5-Carboxyvaleryl)-glycyl]-amino-2-desox-β-D-glucopyranosyl}-N-octadecyl-octadecanamid,
8.a N-{2-[N-(7-Carboxyheptanoyl)-glycyl]-amino-2-desoxy-β-D-glucopyranosyl}-N-dodecyl-octadecanamid,
8.b N-{2-[N-(7-Carboxyheptanoyl)-glycyl]-amino-2-desoxy-β-D-glucopyranosyl}-N-tetradecyl-octadecanamid,
8.c N-{2-[N-(7-Carboxyheptanoyl)-glycyl]-amino-2-desoxy-β-D-glucopyranosyl}-N-octadecyl-dodecanamid,

8.d N-{2-[N-(7-Carboxyheptanoyl)-glycyl]-amino-2-desoxy-β-D-glucopyranosyl}-N-octadecyl-octadecanamid,

9.a N-{2-[N-(3-Carboxypropionyl)-L-alanyl]-amino-2-desoxy-β-D-glucopyranosyl}-N-dodecyl-octadecanamid,

9.b N-{2-[N-(3-Carboxypropionyl)-L-alanyl]-amino-2-desoxy-β-D-glucopyranosyl}-N-tetradecyl-octadecanamid,

9.c N-{2-[N-(3-Carboxypropionyl)-L-alanyl]-amino-2-desoxy-β-D-glucopyranosyl}-N-octadecyl-dodecanamid,

9.a N-{2-[N-(3-Carboxypropionyl)-L-alanyl]-amino-2-desoxy-β-D-glucopyranosyl}-N-octadecyl-octadecanamid,

10.a N-{2-[N-(4-Carboxybutyryl)-L-alanyl]-amino-2-desoxy-β-D-glucopyranosyl}-N-dodecyl-octadecanamid,

10.b N-{2-[N-(4-Carboxybutyryl)-L-alanyl]-amino-2-desoxy-β-D-glucopyranosyl}-N-tetradecyl-octadecanamid,

10.c N-{2-[N-(4-Carboxybutyryl)-L-alanyl]-amino-2-desoxy-β-D-glucopyranosyl}-N-octadecyl-dodecanamid,

10.d N-{2-[N-(4-Carboxybutyryl)-L-alanyl]-amino-2-desoxy-β-D-glucopyranosyl}-N-octadecyl-octadecanamid,

11.a N-{2-[N-(5-Carboxyvaleryl)-L-alanyl]-amino-2-desoxy-β-D-glucopyranosyl}-N-dodecyl-octadecanamid,

11.b N-{2-[N-(4-Carboxyvaleryl)-L-alanyl]-amino-2-desoxy-β-D-glucopyranosyl}-N-tetradecyl-octadecanamid,

11.c N-{2-[N-(4-Carboxyvaleryl)-L-alanyl]-amino-2-desoxy-β-D-glucopyranosyl}-N-octadecyl-dodecanamid,

11.d N-{2-[N-(4-Carboxyvaleryl)-L-alanyl]-amino-2-desoxy-β-D-glucopyranosyl}-N-octadecyl-octadecanamid,

12.a N-{2-[N-(8-Carboxyheptanoyl)-L-alanyl]-amino-2-desoxy-β-D-glucopyranosyl}-N-dodecyl-octadecanamid,

12.b N-{2-[N-(8-Carboxyheptanoyl)-L-alanyl]-amino-2-desoxy-β-D-glucopyranosyl}-N-tetradecyl-octadecanamid,

12.c N-{2-[N-(8-Carboxyheptanoyl)-L-alanyl]-amino-2-desoxy-β-D-glucopyranosyl}-N-octadecyl-dodecanamid,

12.d N-{2-[N-(8-Carboxyheptanoyl)-L-alanyl]-amino-2-desoxy-β-D-glucopyranosyl}-N-octadecyl-octadecanamid,

13.a N-{2-[N-(3-Carboxypropionyl)-L-leucyl]-amino-2-desoxy-β-D-glucopyranosyl}-N-dodecyl-dodecanamid,

13.b N-{2-[N-(3-Carboxypropionyl)-L-leucyl]-amino-2-desoxy-β-D-glucopyranosyl}-N-dodecyl-tetradecanamid,

13.c N-{2-[N-(3-Carboxypropionyl)-L-leucyl]-amino-2-desoxy-β-D-glucopyranosyl}-N-dodecyl-octadecanamid,

13.d N-{2-[N-(3-Carboxypropionyl)-L-leucyl]-amino-2-desoxy-β-D-glucopyranosyl}-N-tetradecyl-dodecanamid,

13.e N-{2-[N-(3-Carboxypropionyl)-L-leucyl]-amino-2-desoxy-β-D-glucopyranosyl}-N-tetradecyl-tetradecanamid,

13.f N-{2-[N-(3-Carboxypropionyl)-L-leucyl]-amino-2-desoxy-β-D-glucopyranosyl}-N-tetradecyl-octadecanamid,

13.g N-{2-[N-(3-Carboxypropionyl)-L-leucyl]-amino-2-desoxy-β-D-glucopyranosyl}-N-octadecyl-dodecanamid,

13.h N-{2-[N-(3-Carboxypropionyl)-L-leucyl]-amino-2-desoxy-β-D-glucopyranosyl}-N-octadecyl-tetradecanamid,

13.i N-{2-[N-(3-Carboxypropionyl)-L-leucyl]-amino-2-desoxy-β-D-glucopyranosyl}-N-octadecyl-octadecanamid,

14.a N-{2-[N-(4-carboxybutyryl)-L-leucyl]-amino-2-desoxy-β-D-glucopyranosyl}-N-dodecyl-octadecanamid,

14.b N-{2-[N-(4-carboxybutyryl)-L-leucyl]-amino-2-desoxy-β-D-glucopyranosyl}-N-tetradecyl-octadecanamid,

14.c N-{2-[N-(4-carboxybutyryl)-L-leucyl]-amino-2-desoxy-β-D-glucopyranosyl}-N-octadecyl-dodecanamid,

14.d N-{2-[N-(4-carboxybutyryl)-L-leucyl]-amino-2-desoxy-β-D-glucopyranosyl}-N-octadecyl-octadecanamid,
15.a N-{2-[N-(5-Carboxyvaleryl)-L-leucyl]-amino-2-desoxy-β-D-glucopyranosyl}-N-dodecyl-octadecanamid,
15.b N-{2-[N-(5-Carboxyvaleryl)-L-leucyl]-amino-2-desoxy-β-D-glucopyranosyl}-N-tetradecyl-octadecanamid,
15.c N-{2-[N-(5-Carboxyvaleryl)-L-leucyl]-amino-2-desoxy-β-D-glucopyranosyl}-N-octadecyl-dodecanamid,
15.d N-{2-[N-(5-Carboxyvaleryl)-L-leucyl]-amino-2-desoxy-β-D-glucopyranosyl}-N-octadecyl-octadecanamid,
16.a N-{2-[N-(7-Carboxyheptanoyl)-L-leucyl]-amino-2-desoxy-β-D-glucopyranosyl}-N-dodecyl-octadecanamid,
16.b N-{2-[N-(7-Carboxyheptanoyl)-L-leucyl]-amino-2-desoxy-β-D-glucopyranosyl}-N-tetradecyl-octadecanamid,
16.c N-{2-[N-(7-Carboxyheptanoyl)-L-leucyl]-amino-2-desoxy-β-D-glucopyranosyl}-N-octadecyl-dodecanamid,
16.d N-{2-[N-(7-Carboxyheptanoyl)-L-leucyl]-amino-2-desoxy-β-D-glucopyranosyl}-N-octadecyl-octadecanamid,
17.a N-{2-[N-(3-Carboxypropionyl)-L-seryl]-amino-2-desoxy-β-D-glucopyranosyl}-N-dodecyl-octadecanamid,
17.b N-{2-[N-(3-Carboxypropionyl)-L-seryl]-amino-2-desoxy-β-D-glucopyranosyl}-N-tetradecyl-octadecanamid,
17.c N-{2-[N-(3-Carboxypropionyl)-L-seryl]-amino-2-desoxy-β-D-glucopyranosyl}-N-octadecyl-dodecanamid,
17.d N-{2-[N-(3-Carboxypropionyl)-L-seryl]-amino-2-desoxy-β-D-glucopyranosyl}-N-octadecyl-octadecanamid,
18.a N-{2-[N-(4-Carboxybutyryl)-L-seryl]-amino-2-desoxy-β-D-glucopyranosyl}-N-dodecyl-octadecanamid,
18.b N-{2-[N-(4-Carboxybutyryl)-L-seryl]-amino-2-desoxy-β-D-glucopyranosyl}-N-tetradecyl-octadecanamid,
18.c N-{2-[N-(4-Carboxybutyryl)-L-seryl]-amino-2-desoxy-β-D-glucopyranosyl}-N-octadecyl-dodecanamid,
18.d N-{2-[N-(4-Carboxybutyryl)-L-seryl]-amino-2-desoxy-β-D-glucopyranosyl}-N-octadecyl-octadecanamid,
19.a N-{2-[N-(5-Carboxyvaleryl)-L-seryl]-amino-2-desoxy-β-D-glucopyranosyl}-N-dodecyl-octadecanamid,
19.b N-{2-[N-(5-Carboxyvaleryl)-L-seryl]-amino-2-desoxy-β-D-glucopyranosyl}-N-tetradecyl-octadecanamid,
19.c N-{2-[N-(5-Carboxyvaleryl)-L-seryl]-amino-2-desoxy-β-D-glucopyranosyl}-N-octadecyl-dodecanamid,
19.d N-{2-[N-(5-Carboxyvaleryl)-L-seryl]-amino-2-desoxy-β-D-glucopyranosyl}-N-octadecyl-octadecanamid,
20.a N-{2-[N-(7-Carboxyheptanoyl)-L-seryl]-amino-2-desoxy-β-D-glucopyranosyl}-N-dodecyl-octadecanamid,
20.b N-{2-[N-(7-Carboxyheptanoyl)-L-seryl]-amino-2-desoxy-β-D-glucopyranosyl}-N-tetradecyl-octadecanamid,
20.c N-{2-[N-(7-Carboxyheptanoyl)-L-seryl]-amino-2-desoxy-β-D-glucopyranosyl}-N-octadecyl-dodecanamid,
20.d N-{2-[N-(7-Carboxyheptanoyl)-L-seryl]-amino-2-desoxy-β-D-glucopyranosyl}-N-octadecyl-octadecanamid,
21.a N-{2-[N-(3-Carboxypropionyl)-L-phenylalanyl]-amino-2-desoxy-β-D-glucopyranosyl}-N-dodecyl-octadecanamid,
21.b N-{2-[N-(3-Carboxypropionyl)-L-phenylalanyl]-amino-2-desoxy-β-D-glucopyranosyl}-N-tetradecyl-octadecanamid,
21.c N-{2-[N-(3-Carboxypropionyl)-L-phenylalanyl]-amino-2-desoxy-β-D-glucopyranosyl}-N-octadecyl-dodecanamid,
21.d N-{2-[N-(3-Carboxypropionyl)-L-phenylalanyl]-amino-2-desoxy-β-D-glucopyranosyl}-N-octadecyl-octadecanamid,

22.a N-{2-[N-(4-Carboxybutyryl)-L-phenylalanyl]-amino-2-desoxy-β-D-glucopyranosyl}-N-dodecyl-octadecanamid,

22.b N-{2-[N-(4-Carboxybutyryl)-L-phenylalanyl]-amino-2-desoxy-β-D-glucopyranosyl}-N-tetradecyl-octadecanamid,

22.c N-{2-[N-(4-Carboxybutyryl)-L-phenylalanyl]-amino-2-desoxy-β-D-glucopyranosyl}-N-octadecyl-dodecanamid,

22.d N-{2-[N-(4-Carboxybutyryl)-L-phenylalanyl]-amino-2-desoxy-β-D-glucopyranosyl}-N-octadecyl-octadecanamid,

23.a N-{2-[N-(5-Carboxyvaleryl)-L-phenylalanyl]-amino-2-desoxy-β-D-glucopyranosyl}-N-dodecyl-octadecanamid,

23.b N-{2-[N-(5-Carboxyvaleryl)-L-phenylalanyl]-amino-2-desoxy-β-D-glucopyranosyl}-N-tetradecyl-octadecanamid,

23.c N-{2-[N-(5-Carboxyvaleryl)-L-phenylalanyl]-amino-2-desoxy-β-D-glucopyranosyl}-N-octadecyl-dodecanamid,

23.d N-{2-[N-(5-Carboxyvaleryl)-L-phenylalanyl]-amino-2-desoxy-β-D-glucopyranosyl}-N-octadecyl-octadecanamid,

24.a N-{2-[N-(7-Carboxyheptanoyl)-L-phenylalanyl]-amino-2-desoxy-β-D-glucopyranosyl}-N-dodecyl-octadecanamid,

24.b N-{2-[N-(7-Carboxyheptanoyl)-L-phenylalanyl]-amino-2-desoxy-β-D-glucopyranosyl}-N-tetradecyl-octadecanamid,

24.c N-{2-[N-(7-Carboxyheptanoyl)-L-phenylalanyl]-amino-2-desoxy-β-D-glucopyranosyl}-N-octadecyl-dodecanamid,

24.d N-{2-[N-(7-Carboxyheptanoyl)-L-phenylalanyl]-amino-2-desoxy-β-D-glucopyranosyl}-N-octadecyl-octadecanamid,

25.a N-[2-(3-Carboxypropionylamino)-2-desoxy-β-D-galactopyranosyl]-N-dodecyl-dodecanamid,

25.b N-[2-(3-Carboxypropionylamino)-2-desoxy-β-D-galactopyranosyl]-N-dodecyl-tetradecanamid,

25.c N-[2-(3-Carboxypropionylamino)-2-desoxy-β-D-galactopyranosyl]-N-dodecyl-octadecanamid,

25.d N-[2-(3-Carboxypropionylamino)-2-desoxy-β-D-galactopyranosyl]-N-tetradecyl-dodecansäure amid,

25.e N-[2-(3-Carboxypropionylamino)-2-desoxy-β-D-galactopyranosyl]-N-tetradecyl-tetradecanamid,

25.f N-[2-(3-Carboxypropionylamino)-2-desoxy-β-D-galactopyranosyl]-N-tetradecyl-octadecanamid,

25.g N-[2-(3-Carboxypropionylamino)-2-desoxy-β-D-galactopyranosyl]-N-octadecyl-dodecanamid,

25.h N-[2-(3-Carboxypropionylamino)-2-desoxy-β-D-galactopyranosyl]-N-octadecyl-tetradecanamid,

25.i N-[2-(3-Carboxypropionylamino)-2-desoxy-β-D-galactopyranosyl]-N-octadecyl-octadecanamid,

26.a N-[2-(4-Carboxybutyrylamino)-2-desoxy-β-D-galactopyranosyl]-N-dodecyl-octadecanamid,

26.b N-[2-(4-Carboxybutyrylamino)-2-desoxy-β-D-galactopyranosyl]-N-tetradecyl-octadecanamid,

26.c N-[2-(4-Carboxybutyrylamino)-2-desoxy-β-D-galactopyranosyl]-N-octadecyl-dodecanamid,

26.d N-[2-(4-Carboxybutyrylamino)-2-desoxy-β-D-galactopyranosyl]-N-octadecyl-octadecanamid,

27.a N-[2-5-Carboxyvalerylamino)-2-desoxy-β-D-galactopyranosyl]-N-dodecyl-octadecanamid,

27.b N-[2-5-Carboxyvalerylamino)-2-desoxy-β-D-galactopyranosyl]-N-tetradecyl-octadecanamid,

27.c N-[2-5-Carboxyvalerylamino)-2-desoxy-β-D-galactopyranosyl]-N-octadecyl-dodecanamid,

27.d N-[2-5-Carboxyvalerylamino)-2-desoxy-β-D-galactopyranosyl]-N-octadecyl-octadecanamid,

28.a N-[2-(7-Carboxyheptanoylamino)-2-desoxy-β-D-galactopyranosyl]-N-dodecyl-ocatdecanamid,

28.b N-[2-(7-Carboxyheptanoylamino)-2-desoxy-β-D-galactopyranosyl]-N-tetradecyl-octadecanamid,

28.c N-[2-(7-Carboxyheptanoylamino)-2-desoxy-β-D-galactopyranosyl]-N-octadecyl-dodecanamid,

28.d N-[2-(7-Carboxyheptanoylamino)-2-desoxy-β-D-galactopyranosyl]-N-octadecyl-octadecanamid,

29.a N-{2-[N-(3-Carboxypropionyl)-glycyl]-amino-2-desoxy-β-D-galactopyranosyl}-N-dodecyl-dodecanamid,

29.b N-{2-[N-(3-Carboxypropionyl)-glycyl]-amino-2-desoxy-β-D-galactopyranosyl}-N-dodecyl-tetradecanamid,

29.c N-{2-[N-(3-Carboxypropionyl)-glycyl]-amino-2-desoxy-β-D-galactopyranosyl}-N-dodecyl-octadecanamid,

29.d N-{2-[N-(3-Carboxypropionyl)-glycyl]-amino-2-desoxy-β-D-galactopyranosyl}-N-tetradecyl-dodecanamid,

29.e N-{2-[N-(3-Carboxypropionyl)-glycyl]-amino-2-desoxy-β-D-galactopyranosyl}-N-tetradecyl-tetradecanamid,

29.f N-{2-[N-(3-Carboxypropionyl)-glycyl]-amino-2-desoxy-β-D-galactopyranosyl}-N-tetradecyl-octadecanamid,

29.g N-{2-[N-(3-Carboxypropionyl)-glycyl]-amino-2-desoxy-β-D-galactopyranosyl}-N-octadecyl-dodecanamid,

29.h N-{2-[N-(3-Carboxypropionyl)-glycyl]-amino-2-desoxy-β-D-galactopyranosyl}-N-octadecyl-tetradecanamid,

29.i N-{2-[N-(3-Carboxypropionyl)-glycyl]-amino-2-desoxy-β-D-galactopyranosyl}-N-octadecyl-octadecanamid.

7. Verfahren zur Herstellung der Verbindungen gemäß den Ansprüchen 1 bis 6, dadurch gekennzeichnet, daß Glykosylamide der allgemeinen Formel (II)

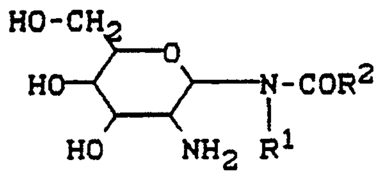

II,

in welcher
$R^1$ und $R^2$ die in den Ansprüchen 1 bis 3 angegebene Bedeutung haben
oder
Peptidoglycolipide der allgemeinen Formel (III)

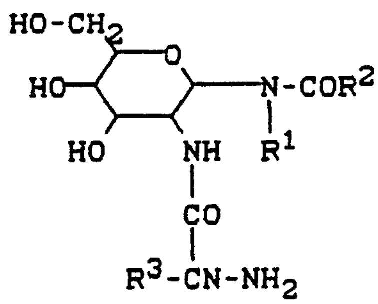

III,

in der
$R^1$, $R^2$ und $R^3$ die in den Ansprüchen 1 bis 5 angegebene Bedeutung haben,
nach bekannten Methoden mit aktivierten Dicarbonsäurederivaten oder mit nicht aktivierten Dicarbonsäurederivaten in Gegenwart eines Kondensationsmittels umsetzt.

8. Arzneimittel enthaltend eine oder mehrere der Verbindungen aus den Ansprüchen 1 bis 6.

9. Verwendung einer der Verbindungen aus den Ansprüchen 1 bis 6 zur Herstellung von Arzneimitteln.

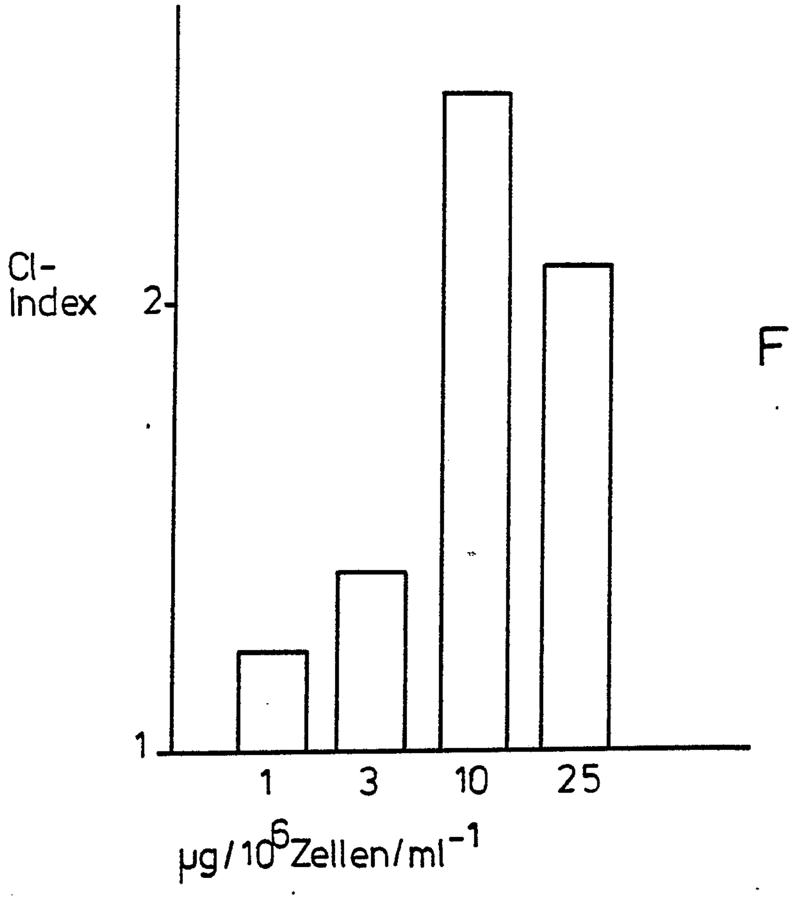


FIG.1a

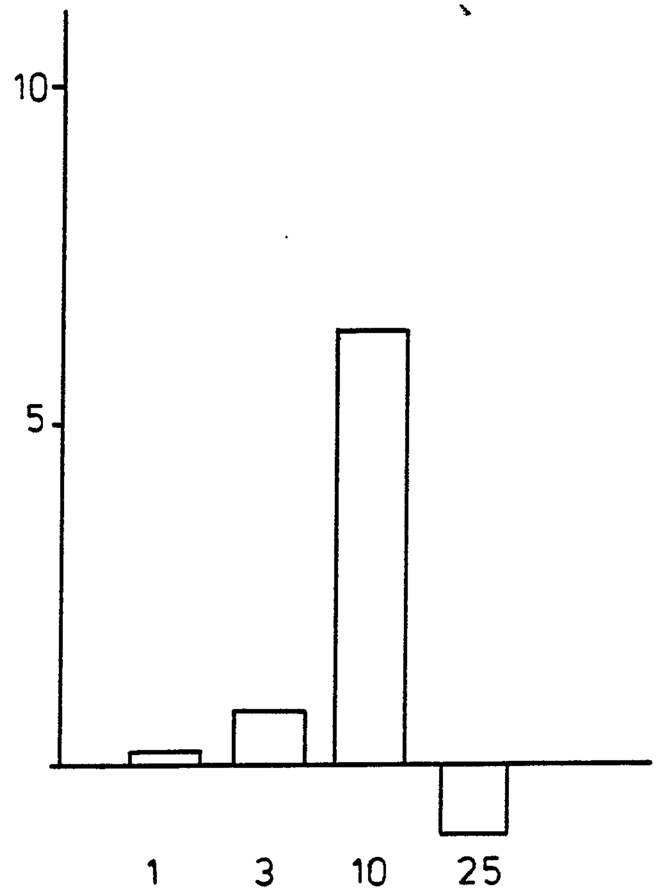


FIG.1b